# EUROPÄISCHE PATENTSCHRIFT

(11) **EP 2 760 399 B1**
(45) Veröffentlichungstag und Bekanntmachung des Hinweises auf die Patenterteilung: **07.12.2016**
(21) Anmeldenummer: 12773224.6
(22) Anmeldetag: 27.09.2012
(51) Int. Cl.: A61F 9/007

(54) **OPHTHALMOCHIRURGISCHE PULSSTEUERUNGSVORRICHTUNG**
OPHTHALMIC SURGICAL PULSE CONTROL DEVICE
DISPOSITIF DE RÉGULATION D'IMPULSIONS EN CHIRURGIE OPHTALMOLOGIQUE

(30) Priorität: 29.09.2011 DE 102011114524
(43) Veröffentlichungstag der Anmeldung: 06.08.2014
(73) Patentinhaber: Carl Zeiss Meditec AG, 07745 Jena (DE)
(72) Erfinder: KUEBLER, Christoph, 73447 Oberkochen (DE); EICHLER, Michael, 73434 Aalen (DE)
(74) Vertreter: Carl Zeiss AG - Patentabteilung
(86) Internationale Anmeldenummer: PCT/EP2012/004054
(87) Internationale Veröffentlichungsnummer: WO 2013/045093

(56) Entgegenhaltungen:
- EP-A1- 1 759 672
- WO-A2-01/41672
- WO-A2-2008/016870

## Beschreibung

Die Erfindung betrifft eine ophthalmochirurgische Pulssteuerungsvorrichtung und ein ophthalmochirurgisches System mit einer solchen Pulssteuerungsvorrichtung.

Zur Behandlung einer Linsentrübung, welche in der Medizin als grauer Star bezeichnet wird, gibt es mehrere chirurgische Techniken. Die am weitesten verbreitete Technik ist die Phakoemulsifikation, bei der eine dünne Nadel in die erkrankte Linse eingeführt und mit Ultraschall zu Schwingungen angeregt wird. Die vibrierende Nadel emulsifiziert in ihrer nächsten Umgebung die Linse derart, dass die entstehenden Linsenfragmente durch eine Leitung von einer Pumpe abgesaugt werden können. Ist die Linse vollständig emulsifiziert worden, kann in den leeren Kapselsack eine neue künstliche Linse eingesetzt werden, so dass ein derart behandelter Patient wieder ein gutes Sehvermögen erreichen kann.

Das Zertrümmern einer erkrankten Linse durch eine mit Ultraschall schwingende Nadel funktioniert in der Praxis recht gut. Je höher die der Nadel zugeführte Energie und je länger die Ultraschallschwingung andauert, umso schneller können von einer Linse kleine Partikel erzeugt und diese anschließend abgesaugt werden. Nachteilig ist dabei jedoch, dass bei einem solchen hohen Energieeintrag in der Umgebung der schwingenden Nadel eine relativ hohe Temperatur entsteht. Da die Nadel für die Operation durch die Hornhaut gestochen werden muss, kann dies zu einer Verbrennung der Hornhaut führen, was unbedingt vermieden werden muss. Ferner können bei einem hohen Energieeintrag mit einer großen Amplitude der Nadelschwingung kleine Linsenpartikel von der Nadelspitze fortgestoßen werden. Die Schwingungsenergie der Nadel wird somit in eine Bewegungsenergie von kleinen Partikeln umgesetzt, anstatt diese zu zerkleinern und abzusaugen. Dies führt ebenfalls zu einer Erhöhung der Temperatur im Auge. Eine solche Temperaturerhöhung lässt sich zwar umgehen, indem mit einer relativ niedrigen Ultraschallenergie gearbeitet wird. Dabei verlängert sich jedoch die Operationsdauer deutlich. Zudem können relativ große und harte Partikel nicht zerkleinert werden.

In der EP 1 759 672 A1 ist ein Verfahren zur Beeinflussung von Form, Sequenz und Dauer von Ultraschallenergie-Pulsen für ein Ultraschall-Handstück eines ophthalmochirurgischen Systems beschrieben.

In der WO 01/41672 A2 ist eine Phakoemulsifikationsvorrichtung beschrieben, die dazu eingerichtet ist, ein Ultraschall-Handstück in einem Zufalls-Puls-Modus zu steuern.

Es besteht somit eine Aufgabe, eine ophthalmochirurgische Pulssteuerungsvorrichtung zu schaffen, mit der eine Nadel eines Phakoemulsifikations-Handstückes so angesteuert werden kann, dass möglichst viele Partikel bei geringem Energieeintrag und kurzer Operationsdauer erzeugt und abgesaugt werden können.

Die Aufgabe wird durch den Gegenstand des unabhängigen Patentanspruchs gelöst. Vorteilhafte Weiterbildungen der Erfindung sind Gegenstand der Unteransprüche.

Die ophthalmochirurgische Pulssteuerungsvorrichtung gemäß der Erfindung weist einen Pulsgenerator auf, welcher eingerichtet ist, während einer Einschaltdauer des Pulsgenerators Pulse mit einer Pulsdauer zu erzeugen, in welcher die Nadel eines Phakoemulsifikations-Handstückes im Wesentlichen in Resonanz schwingt, und welcher eingerichtet ist, während der Einschaltdauer des Pulsgenerators Pulspausen mit einer Pulspausendauer zu erzeugen, in denen die Nadel nur minimal oder gar nicht schwingt, wobei ein Puls mit einer darauf folgenden Pulspause ein Pulspaket mit einer Pulspaketdauer bildet, wobei der Pulsgenerator eingerichtet ist, unmittelbar aufeinanderfolgende Pulspakete zu erzeugen, wobei die Folge der Beträge von Pulsdauer und Pulspausendauer von aufeinanderfolgenden Pulspaketen während der gesamten Einschaltdauer des Pulsgenerators eine aperiodische Folge bildet.

Eine solche Aperiodizität einer Folge der Beträge aus Pulsdauer und Pulspausendauer führt. dazu, dass während der gesamten Einschaltdauer des Pulsgenerators keine Wiederholung eines Pulsmusters auftritt. Eine Periodendauer oder eine Frequenz eines Pulsmusters liegt nicht vor. Dies bewirkt, dass sowohl kleine als auch große Linsenpartikel mit sowohl hoher als auch niedriger Härte gut zertrümmert und abgesaugt werden können. Wenn eine Linse nur zum Teil eine harte Zone besitzt, ist es somit nicht erforderlich, die Operation zur Sicherheit mit einer hohen Energie bei einer sich wiederholenden Folge der Beträge aus Pulsdauer mit jeweils sich anschließender Pulspausendauer durchzuführen. Durch die aperiodische Folge der Beträge aus Pulsdauer und Pulspausendauer von aufeinanderfolgenden Pulspaketen gibt es während der gesamten Einschaltdauer des Pulsgenerators genügend Pulse, welche zum Beispiel nur kurz andauern und eine geringe Energie ins Auge einbringen, so dass auch weiche Linsenteile zerkleinert und emulsifiziert werden können. Ferner sind auch Pulse dabei, welche länger andauern und mit einem höheren Energieeintrag verbunden sind, so dass auch große und harte Partikel zertrümmert werden können. Die Aperiodizität führt ferner dazu, dass sich keine stehenden Wellen relativ zur Spitze der Nadel ausbilden können, so dass keine zusätzliche lokale Erwärmung erzeugt wird. Ferner sind bei einer aperiodischen Folge der Beträge aus Pulsdauer und Pulspausendauer genügend Pulspakete vorhanden, bei denen kleine Partikel von der Spitze der Phakoemulsifikationsnadel nicht fortgestoßen werden, sondern sich gut absaugen lassen. Somit lassen sich durch die erfindungsgemäße Pulssteuerungsvorrichtung viele Partikel mit unterschiedlichen Eigenschaften bei geringem Energieeintrag und kurzer Operationsdauer erzeugen und absaugen.

Vorzugsweise sind die Beträge von Pulsdauer und Pulspausendauer eines Pulspaketes von den Beträgen von Pulsdauer und Pulspausendauer eines unmittelbar nachfolgenden Pulspaketes verschieden. Damit ist sichergestellt, dass nie zwei Pulspakete mit den gleichen Pulszeiten aufeinander folgen, so dass sich auch nicht kurzzeitig eine Wiederholung von Pulspaketen ergibt.

Gemäß einer weiteren Ausführungsform tritt in der aperiodischen Folge der Beträge aus Pulsdauer und Pulspausendauer eine Pulsdauer und eine Pulspausendauer jeweils nur ein einziges Mal auf. Damit kann eine Folge der Beträge von Pulsdauern oder eine Folge der Beträge von Pulspausendauern mit einem linear ansteigenden, einem linear abfallenden, einem logarithmischen oder exponentiellen Verlauf erreicht werden. Dies ermöglicht einen linear ansteigenden, linear abfallenden, logarithmischen oder exponentiellen Energieeintrag, welches für die Emulsifikation von Linsen mit ganz unterschiedlichen Härtebereichen vorteilhaft sein kann. Bei einem zum Beispiel exponentiell ansteigenden Verlauf der Pulsdauern und exponentiell ansteigenden Verlauf der Pulspausendauern kann anfangs mit kurzen Pulspaketen ein sehr intensives Zertrümmern von Linsenteilen durchgeführt werden, wobei mit zunehmender Zeitdauer die Pulsdauern und Pulspausendauern zunehmen. Mit zunehmender Zeitdauer verlängert sich damit auch die Zeit, die zur Abkühlung zur Verfügung gestellt wird.

Erfindungsgemäß variieren die Beträge der Pulsdauer aufeinanderfolgender Pulspakete um einen vorbestimmten Mittelwert mit einer vorbestimmten positiven und negativen Abweichung dazu. Wenn ein Operateur aufgrund einer Voruntersuchung weiß, dass bei einem zum Beispiel älteren Patienten eine Linse mit einer relativ hohen Härte vorliegt, kann er den Mittelwert einer durchschnittlichen Pulsdauer so einstellen, dass voraussichtlich genügend Energie zur Verfügung steht, um die harte Linse zu emulsifizieren. Wenn jedoch bei einer Voruntersuchung festgestellt wird, dass der Patient eine sehr weiche Linse hat, kann der Mittelwert der Pulsdauer von aufeinanderfolgenden Pulspaketen auf einen niedrigen Wert gesetzt werden, so dass noch relativ wenig Energie zugeführt wird. Damit wird vermieden, dass unnötig viel Energie und damit Wärme in das Auge eingebracht wird.

Vorzugsweise ist das Verhältnis der Beträge von Pulsdauer zu Pulspausendauer auf einen vorbestimmten Wert einstellbar, welche größer als 0,01 ist. Dies begrenzt die Folge der Beträge aus Pulsdauer und Pulspausendauer derart, dass immer ein Mindestbetrag an Energie zugeführt wird.

Die Pulssteuerungsvorrichtung kann auch derart ausgebildet sein, dass die Zahl der Pulse je Zeiteinheit einstellbar ist. Bei einer harten Linse kann eine relativ hohe Zahl an Pulsen je Zeiteinheit verwendet werden, wohingegen bei einer weichen Linse mit einer niedrigen Pulszahl je Zeiteinheit gearbeitet werden kann.

Bei der Pulssteuerungsvorrichtung kann sich eine dem Phakoemulsifikations-Handstück zugeführte Energie während der Pulsdauer eines Pulspaketes von einer zugeführten Energie während der Pulsdauer eines unmittelbar nachfolgenden Pulspaketes unterscheiden.

Dies erhöht noch einmal die Variabilität der Vorrichtung. Falls eine Linse mit sehr harten Bereichen, aber auch sehr weichen Bereichen vorliegt, kann damit eine besonders kurze Operationsdauer erzielt werden.

Die Aufgabe wird auch durch ein ophthalmochirurgisches System gelöst, welche eine Pulssteuerungsvorrichtung wie vorstehend beschrieben aufweist, und zudem eine Fluidsteuerungsvorrichtung, eine Energieversorgung, ein Phakoemulsifikations-Handstück, eine Eingabeeinheit und eine zentrale Steuerungseinheit aufweist, welche mit der Pulssteuerungsvorrichtung, der Fluidsteuerungsvorrichtung, der Energieversorgung, dem Phakoemulsifikation-Handstück und der Eingabeeinheit gekoppelt ist.

Weitere Vorteile und Merkmale der Erfindung werden mit Bezug auf die nachfolgenden Zeichnungen erklärt, in welchen zeigen:
- Figur 1: eine Folge aus Pulsdauer und Pulspausendauer von aufeinanderfolgenden Pulspaketen;
- Figur 2: ein Diagramm, welches für die Pulspakete gemäß Fig. 1 die jeweilige Dauer der Pulse und Pulspausen darstellt;
- Figur 3: ein Diagramm, welches die Pulsdauer von aufeinander folgenden Pulspaketen in Abhängigkeit von der Zeit darstellt;
- Figur 4: ein Diagramm, welches die zugeführte Energie von aufeinanderfolgenden Pulspaketen in Abhängigkeit von der Zeit darstellt;
- Figur 5: eine weitere Folge aus Pulsdauer und Pulspausendauer von aufeinanderfolgenden Pulspaketen;
- Figur 6: ein Diagramm, welches für die Pulspakete gemäß Fig. 5 die jeweilige Dauer der Pulse und Pulspausen darstellt;
- Figur 7: eine weitere Folge aus Pulsdauer und Pulspausendauer von aufeinanderfolgenden Pulspaketen;
- Figur 8: ein Diagramm, welches für die Pulspakete gemäß Fig. 7 die jeweilige Dauer der Pulse und Pulspausen darstellt; und
- Figur 9: eine schematische Darstellung eines ophthalmochirurgischen Systems gemäß der Erfindung.

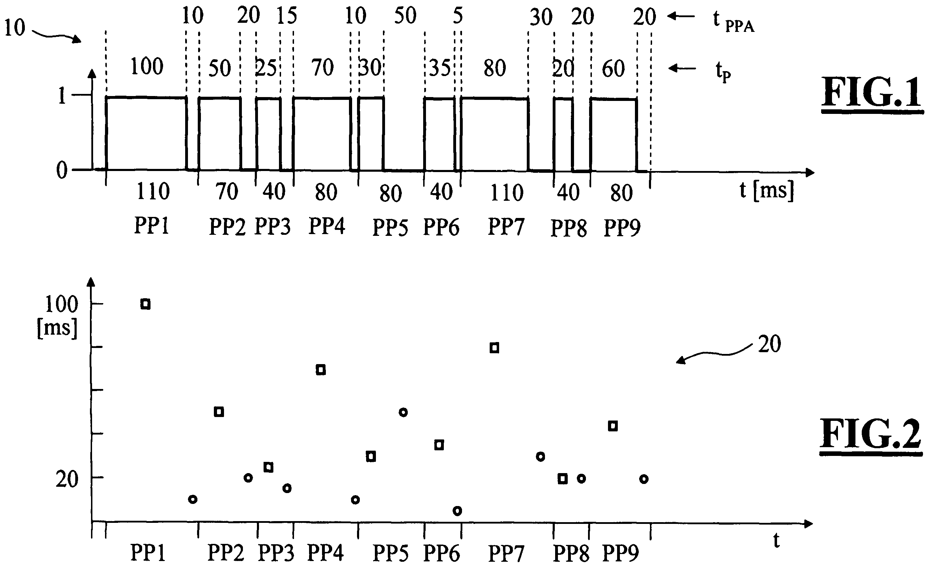

In Figur 1 ist ein Diagramm 10 dargestellt, welches eine Folge aus Pulsdauer und Pulspausendauer von aufeinanderfolgenden Pulspaketen PP zeigt. Die Ordinate zeigt den Zustand "keine Pulse" mit "0" und den Zustand "Pulse eingeschaltet" mit "1" an. Das erste Pulspaket PP1 besitzt eine Pulspaketdauer von zum Beispiel 110 Millisekunden (ms), wobei die Pulsdauer 100 ms und die Pulspausendauer 10 ms beträgt. Unmittelbar darauf folgt ein zweites Pulspaket PP2 mit einer Pulspaketdauer von 70 ms, wobei die Pulsdauer 50 ms und die Pulspausendauer 20 ms beträgt. Daraufhin folgt ein drittes Pulspaket PP3 mit einer Pulspaketdauer von 40 ms, wobei die Pulsdauer 25 ms und die Pulspausendauer 15 ms beträgt. Figur 1 zeigt weitere darauf folgende Pulspakete PP4 bis PP9 mit den jeweiligen Pulspaketdauern und der jeweiligen Pulsdauer und Pulspausendauer. Somit ergibt sich eine Folge der Beträge aus Pulsdauer und Pulspausendauer wie folgt (Zahlenangaben in Millisekunden):
100, 10, 50, 20, 25, 15, 70, 10, 30, 50, 35, 5, 80, 30, 20, 20, 60, 20.

Wie leicht zu erkennen ist, ist diese Folge der Beträge aus Pulsdauer und Pulspausendauer von aufeinanderfolgenden Pulspaketen eine aperiodische Folge. Ein regelmäßiges Muster ist nicht vorhanden, eine Schwingung mit einer Periode existiert nicht.

Bei der beschriebenen Folge liegen während der gesamten Einschaltdauer des Pulsgenerators somit insgesamt 9 Pulspakete an. Anschließend ist der Pulsgenerator abgeschaltet. Nach dem erneuten Einschalten bewirkt die erfindungsgemäße Pulssteuerungsvorrichtung , dass wieder während der gesamten Einschaltdauer des Pulsgenerators eine aperiodische Folge der Beträge aus Pulsdauer und Pulspausendauer anliegt.

In Figur 2 ist ein Diagramm 20 gezeigt, bei dem auf der Ordinatenachse die Dauer der Pulse und Pulspausen und auf der Abszissenachse die jeweiligen in Fig. 1 angegebenen Pulspakete aufgetragen sind. Die jeweilige Pulsdauer ist durch ein quadratisches Symbol dargestellt, während die jeweilige Pulspausendauer durch ein Kreissymbol dargestellt ist. Aus dem Diagramm ist deutlich ersichtlich, dass während der gesamten Einschaltdauer des Pulsgenerators eine aperiodische Folge der Beträge aus Pulsdauer und Pulspausendauer vorliegt.

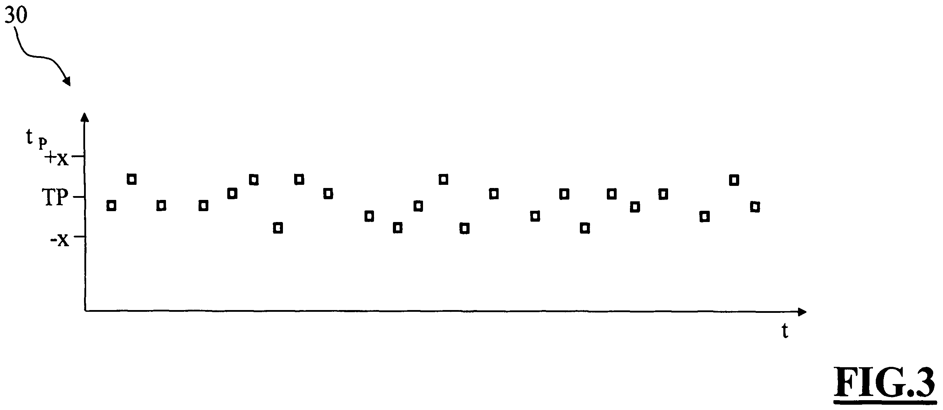

In Figur 3 ist ein Diagramm 30 dargestellt, welches die Beträge der Pulsdauer von aufeinander folgenden Pulspaketen zeigt. Die Beträge der Pulsdauer von aufeinanderfolgenden Pulspakten variiert dabei um einen vorbestimmten Mittelwert TP mit einer vorbestimmten positiven Abweichung +x und negativen Abweichung -x. Diese aperiodische Folge zeigt, dass die Pulsdauer nie auf ganz niedrige Werte absinkt. Dies ist zum Beispiel sinnvoll, wenn eine relativ harte Linse zu emulsifizieren ist, so dass der Ophthalmologe schon vor Beginn der Operation weiß, dass nur mit relativ langer Pulsdauer gearbeitet werden soll.

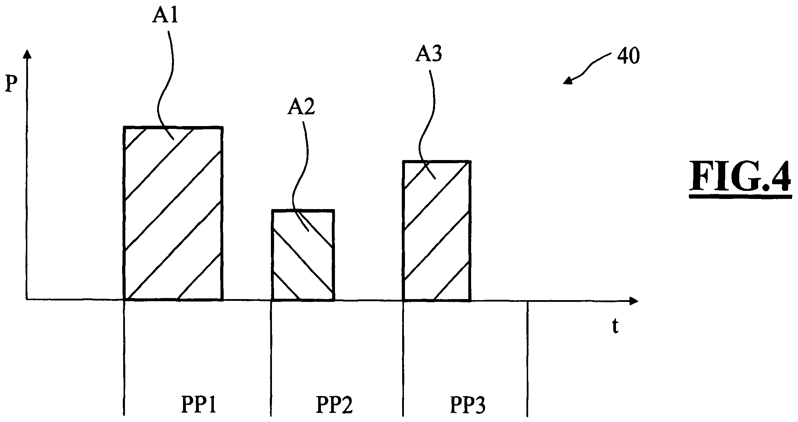

In Figur 4 ist ein Diagramm 40 dargestellt, welches die zugeführte Energie P von aufeinander folgenden Pulspaketen PP1 bis PP3 in Abhängigkeit von der Zeit darstellt. Die Fläche A1 bedeutet eine relativ hohe Energie, wohingegen die nachfolgende Fläche A2 während einer nachfolgenden Pulsdauer eine relativ niedrige Energie anzeigt. Das dritte Pulspaket zeigt eine Pulsdauer mit einer zugeführten Energie gemäß der mit A3 schraffierten Fläche, welche sich von der Fläche A1 und A2 unterscheidet. Die Variation in der aperiodischen Folge der Beträge von einzelnen Pulsdauern und Pulspausendauern kann durch die unterschiedlich zugeführte Energie zusätzlich erhöht werden.

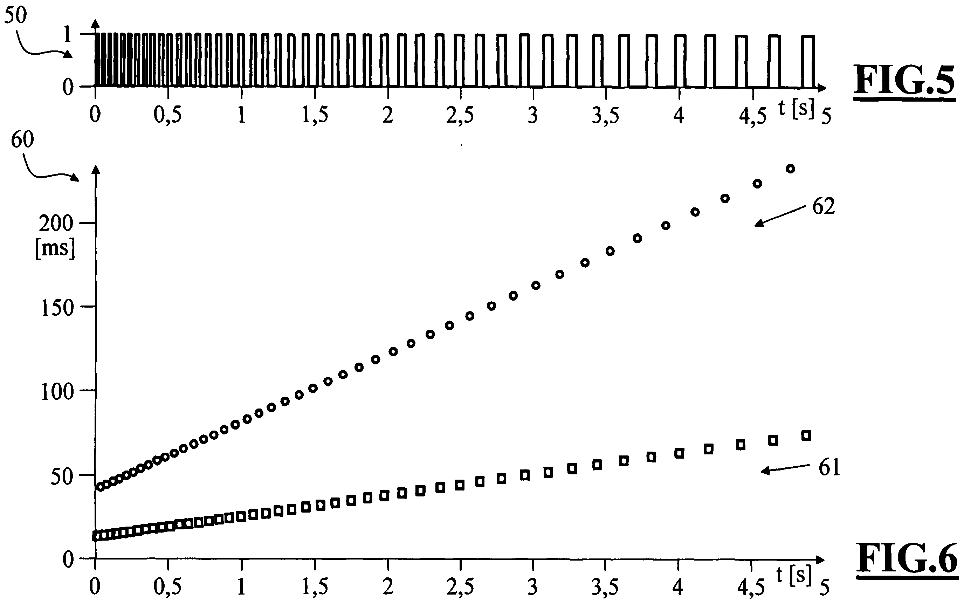

In Figur 5 ist ein Diagramm 50 dargestellt, welches eine Folge aus Pulsdauer und Pulspausendauer von aufeinanderfolgenden Pulspaketen zeigt. Die gesamte Einschaltdauer des Pulsgenerators beträgt bei diesem Beispiel 5 Sekunden. Aus dem zugehörigen Diagramm 60 in Fig. 6 ist ersichtlich, dass die Folge der Beträge von Pulsdauern, siehe Bezugszeichen 61, und die Folge der Beträge der Pulspausendauern, siehe Bezugszeichen 62, der jeweiligen Pulspakete linear zunehmen. Der Betrag einer Pulsdauer eines Pulspaketes unterscheidet sich somit von dem Betrag der nachfolgenden Pulsdauer eines nachfolgenden Pulspaketes. Dies gilt in gleicher Weise für die aufeinander folgenden Pulspausendauern. Das Verhältnis aus einem Betrag einer Pulsdauer zum Betrag einer anschließenden Pulspausendauer eines Pulspaketes unterscheidet sich vom Verhältnis aus einem Betrag einer Pulsdauer zum Betrag einer anschließenden Pulspausendauer eines unmittelbar darauf folgenden Pulspaketes.

Bei dem in Fig. 6 dargestellten Beispiel nimmt mit zunehmender Zahl der Pulspakete die Pulspausendauer zu, was aus medizinischer Sicht sinnvoll ist. Damit wird bei einer ohne Unterbrechung mit Pulspaketen beaufschlagten Nadel eines Phako-Handstückes die Abkühlzeit mit zunehmender Operationsdauer immer länger. Dadurch wird zum Beispiel vermieden, dass es durch die schwingende Nadel zu einer Verbrennung der Hornhaut kommt.

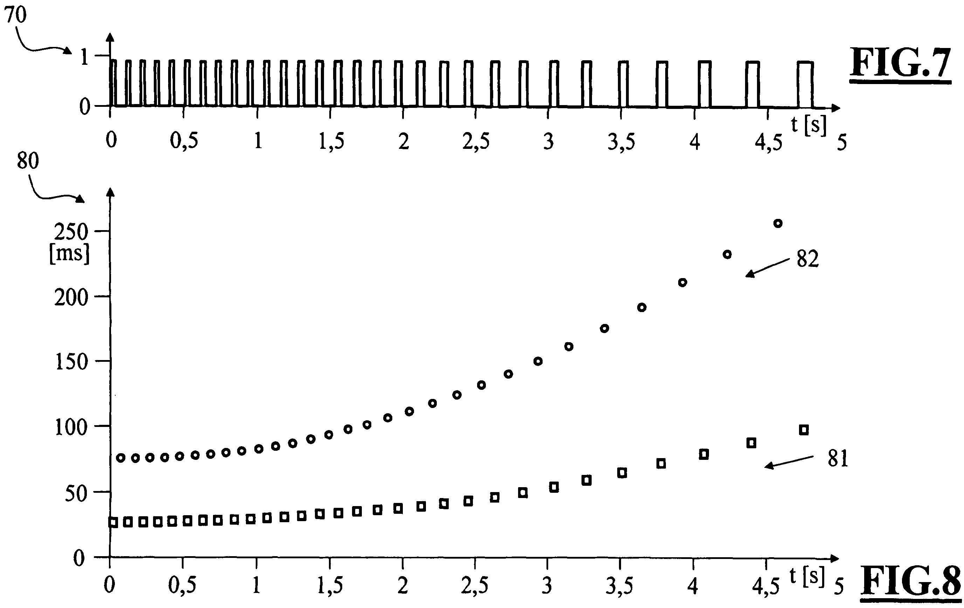

Ein weiteres Beispiel ist in Figur 7 mit einem Diagramm 70 dargestellt, welches eine Folge der Beträge aus Pulsdauer und Pulspausendauer von aufeinanderfolgenden Pulspaketen zeigt. In Figur 8 sind für die Pulse und Pulspausen gemäß Fig. 7 die zugehörigen Zeitdauern eingetragen. Aus Figur 8 ist zu erkennen, dass die Folge der Beträge von Pulsdauern, siehe Bezugszeichen 81, und die Folge der Beträge von Pulspausendauern, siehe Bezugszeichen 82, der jeweiligen Pulspakete exponentiell zunehmen. Auch hier unterscheidet sich ein Betrag einer Pulsdauer eines Pulspaketes von dem Betrag einer nachfolgenden Pulsdauer eines nachfolgenden Pulspaketes. Dies gilt ebenfalls für die aufeinander folgenden Pulspausendauern.

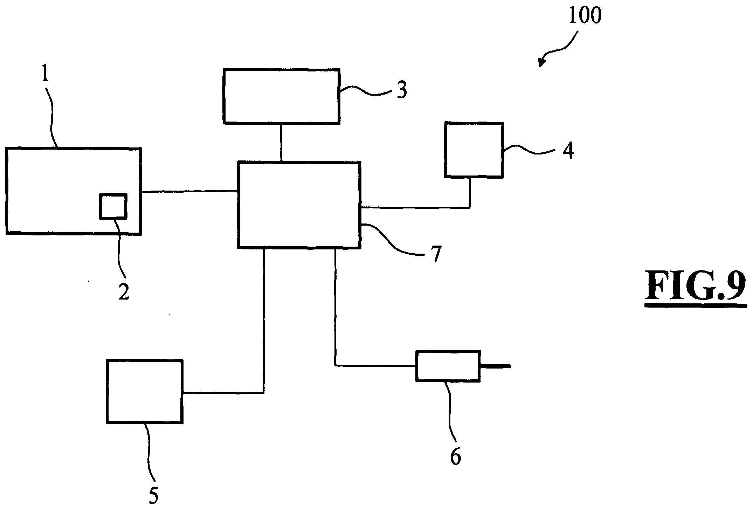

In Figur 9 ist eine schematische Darstellung eines ophthalmochirurgischen Systems 100 dargestellt, welches eine Pulssteuerungsvorrichtung 1 mit einem Pulsgenerator 2, eine Fluidsteuerungsvorrichtung 3, eine Energieversorgung 4, eine Eingabeeinheit 5, ein Phakoemulsifikations-Handstück 6 und eine zentrale Steuerungsvorrichtung 7, welche die genannten Komponenten miteinander verbindet, aufweist.

## Patentansprüche

1. Ophthalmochirurgische Pulssteuerungsvorrichtung, aufweisend einen Pulsgenerator,
- welcher eingerichtet ist, während einer Einschaltdauer des Pulsgenerators Pulse mit einer Pulsdauer zu erzeugen, in welcher die Nadel eines Phakoemulsifikations-Handstückes im Wesentlichen in Resonanz schwingt, und
- welcher eingerichtet ist, während der Einschaltdauer des Pulsgenerators Pulspausen mit einer Pulspausendauer zu erzeugen, in denen die Nadel nur minimal oder gar nicht schwingt,
- wobei ein Puls mit einer darauf folgenden Pulspause ein Pulspaket mit einer Pulspaketdauer bildet,
- wobei der Pulsgenerator eingerichtet ist, unmittelbar aufeinander folgende Pulspakete zu erzeugen,
- wobei die Folge der Beträge von Pulsdauer und Pulspausendauer von aufeinander folgenden Pulspaketen während der gesamten Einschaltdauer des Pulsgenerators eine aperiodische Folge ist,
**dadurch gekennzeichnet, daß**
die Beträge der Pulsdauer aufeinander folgender Pulspakete um einen vorbestimmten Mittelwert mit einer vorbestimmten positiven und negativen Abweichung dazu variieren.

2. Pulssteuerungsvorrichtung nach Anspruch 1, wobei jeweils die Beträge von Pulsdauer und Pulspausendauer eines Pulspaketes von den Beträgen einer Pulsdauer und Pulspausendauer eines unmittelbar nachfolgenden Pulspaketes verschieden sind.

3. Pulssteuerungsvorrichtung nach einem der vorherigen Ansprüche, wobei in der aperiodischen Folge der Beträge aus Pulsdauer und Pulspausendauer eine Pulsdauer und eine Pulspausendauer jeweils nur ein einziges Mal auftritt.

4. Pulssteuerungsvorrichtung nach einem der vorherigen Ansprüche, wobei ein Verhältnis der Beträge von Pulsdauer zu Pulspausendauer auf einen vorbestimmten Wert, vorzugsweise größer als 0,01, einstellbar ist.

5. Pulssteuerungsvorrichtung nach einem der vorherigen Ansprüche, wobei die Zahl der Pulse je Zeiteinheit einstellbar ist.

6. Pulssteuerungsvorrichtung nach einem der vorherigen Ansprüche, wobei sich eine dem Phakoemulsifikations-Handstück zuführbare Energie während der Pulsdauer eines Pulspaketes von einer zuführbaren Energie während der Pulsdauer eines unmittelbar nachfolgenden Pulspaketes unterscheidet.

7. Ophthalmochirurgisches System, aufweisend eine Pulssteuerungsvorrichtung nach einem der vorstehenden Ansprüche, eine Fluidsteuerungsvorrichtung, eine Energieversorgung, ein Phakoemulsifikations-Handstück, eine Eingabeeinheit und eine zentrale Steuerungseinheit, welche mit der Pulssteuerungsvorrichtung, der Fluidsteuerungsvorrichtung, der Energieversorgung, dem Phakoemulsifikations-Handstück und der Eingabeeinheit gekoppelt ist.

## Claims

1. Ophthalmic surgical pulse control device, comprising a pulse generator,
- which, during a switch-on duration of the pulse generator, is configured to produce pulses with a pulse duration during which the needle of a phacoemulsification handpiece substantially vibrates in resonance, and
- which, during the switch-on duration of the pulse generator, is configured to produce pulse pauses with a pulse pause duration during which the needle vibrates only minimally or not at all,
- wherein a pulse with a subsequent pulse pause forms a pulse packet with a pulse packet duration,
- wherein the pulse generator is configured to produce pulse packets which immediately follow one another,
- wherein the sequence of the absolute values of pulse duration and pulse pause duration of successive pulse packets during the entire switch-on duration of the pulse generator is an aperiodic sequence,
**characterized in that** the absolute values of the pulse duration of successive pulse packets vary around a predetermined mean value with a predetermined positive and negative deviation therefrom.

2. Pulse control device according to Claim 1, wherein the absolute values of pulse duration and pulse pause duration of a pulse packet in each case are different from the absolute values of a pulse duration and pulse pause duration of an immediately following pulse packet.

3. Pulse control device according to one of the preceding claims, wherein a pulse duration and a pulse pause duration respectively only occur a single time during the aperiodic sequence of the absolute values of pulse duration and pulse pause duration.

4. Pulse control device according to one of the preceding claims, wherein a ratio of the absolute values of pulse duration to pulse pause duration can be set to a predetermined value, preferably greater than 0.01.

5. Pulse control device according to one of the preceding claims, wherein the number of pulses per unit time can be set.

6. Pulse control device according to one of the preceding claims, wherein energy that may be supplied to the phacoemulsification handpiece during the pulse duration of a pulse packet differs from energy that may be supplied during the pulse duration of an immediately following pulse packet.

7. Ophthalmic surgical system, comprising a pulse control device according to one of the preceding claims, a fluid control device, a power supply, a phacoemulsification handpiece, an input unit and a central control unit, which is coupled to the pulse control device, the fluid control device, the power supply, the phacoemulsification handpiece and the input unit.

## Revendications

1. Dispositif de commande impulsionnelle en chirurgie ophtalmologique, comprenant un générateur d'impulsions,
- qui est conçu pour générer, pendant une période d'activité du générateur d'impulsions, des impulsions ayant une durée d'impulsion pendant laquelle l'aiguille d'une pièce à main de phacoémulsification oscille sensiblement en résonnance, et
- qui est conçu pour générer, pendant la durée d'activation du générateur d'impulsions, des pauses d'impulsions ayant une durée de pause d'impulsion pendant laquelle l'aiguille n'oscille que de manière minimale ou nulle,
- dans lequel une impulsion forme avec une pause d'impulsion qui la suit un paquet d'impulsion ayant une durée de paquet d'impulsion,
- dans lequel le générateur d'impulsions est conçu pour générer des paquets d'impulsions immédiatement consécutifs,
- dans lequel la séquence des valeurs des durées d'impulsions et des durées de pauses d'impulsions de paquets d'impulsions consécutifs pendant la totalité de la durée d'activation du générateur d'impulsions est une séquence apériodique,
**caractérisé en ce que** les valeurs des durées d'impulsions consécutives varient autour d'une valeur moyenne prédéterminée avec des excursions positive et négative prédéterminées par rapport à celle-ci.

2. Dispositif de commande impulsionnelle selon la revendication 1, dans lequel les valeurs de la durée d'impulsion et de la durée de pause d'impulsion d'un paquet d'impulsion sont différentes des valeurs d'une durée d'impulsion et d'une durée de pause d'impulsion d'un paquet d'impulsion immédiatement consécutif.

3. Dispositif de commande impulsionnelle selon l'une quelconque des revendications précédentes, dans lequel, pendant la séquence apériodique des valeurs constituées de la durée d'impulsion et de la durée de pause d'impulsion, une durée d'impulsion et une durée de pause d'impulsion n'apparaissent respectivement qu'une seule fois.

4. Dispositif de commande impulsionnelle selon l'une quelconque des revendications précédentes, dans lequel un rapport des valeurs de la durée d'impulsion à la durée de la pause d'impulsion peut être réglé à une valeur prédéterminée, de préférence supérieure à 0,01.

5. Dispositif de commande impulsionnelle selon l'une quelconque des revendications précédentes, dans lequel le nombre des impulsions par unité de temps peut être réglé.

6. Dispositif de commande impulsionnelle selon l'une quelconque des revendications précédentes, dans lequel une énergie pouvant être délivrée à la pièce à main de phacoémulsification pendant la durée d'impulsion d'un paquet d'impulsion est différente d'une énergie pouvant être délivrée pendant la durée d'impulsion d'un paquet d'impulsion immédiatement consécutif.

7. Système de chirurgie ophtalmologique, comprenant un dispositif de commande impulsionnelle selon l'une quelconque des revendications précédentes, un dispositif de commande fluidique, une alimentation en énergie, une pièce à main de phacoémulsification, une unité d'entrée et une unité centrale de commande qui est couplée au dispositif de commande impulsionnelle, au dispositif de commande fluidique, à l'alimentation en énergie, à la pièce à main de phacoémulsification et à l'unité d'entrée.
